# EUROPEAN PATENT SPECIFICATION

(11) **EP 2 866 009 B1**
(45) Date of publication and mention of the grant of the patent: **03.01.2024**
(21) Application number: 14189947.6
(22) Date of filing: 05.11.2008
(51) Int. Cl.: G01J 5/02, G01J 5/00, G16H 10/60, G16H 40/63, G16H 40/67

(54) **Non-invasive medical data collecting assembly**
Anordnung zum nichtinvasiven Sammeln medizinischer Daten
Ensemble de collecte de données médicales non invasive

(30) Priority: 09.11.2007 US 983613
(43) Date of publication of application: 29.04.2015
(62) Divisional of application: 08847629.6
(73) Proprietor: VISIOMED GROUP, 75016 Paris (FR)
(72) Inventor: Sebban, Eric, 75020 Paris (FR)
(74) Representative: Cabinet Nuss

(56) References cited:
- GB-A- 2 380 791
- JP-A- 2002 202 202
- US-A1- 2002 016 553
- US-A1- 2002 186 317

## Description

### BACKGROUND OF THE INVENTION

### Field of the Invention

The present invention is directed to a non-invasive medical data collecting assembly structured to collect temperature and/or other medical data utilizing a hand-held device operatively disposed relative to a patient. A memory assembly, a display assembly and a control assembly are all operatively interconnected and cooperatively structured to facilitate the storage of medical data from a plurality of different patients which may be selectively and repeatedly accessed, displayed and stored. Data transmitting capabilities are provided to transmit collected medical data from the memory assembly to a remote processing facility.

### DESCRIPTION OF THE RELATED ART

The human body, as well as animals and other objects, whether solid, liquid or gas, release energy by radiation, wherein the strength of the radiation is indicative of the temperature of the object. As such, medical data such as the temperature of a human or animal patient can be acquired by a determination of the strength of the radiation emanating from the patient.

The method of temperature measurements by use of heat radiation possesses many advantages and overcomes certain disadvantages and problems associated with more conventional thermometers or like temperature acquiring devices. As such, the methods for measuring temperature can be classified into two categories including, contact and non-contact type devices. In the case of the contact-type of thermometer or temperature measuring device, heat is transferred directly to the temperature measuring instrument through a contact surface or interface by means of physically engaging the device or a specific portion thereof with a target area of the patient being examined. In contrast, a non-invasive thermometer or temperature acquiring assembly may be disposed a spaced distance from the target area of the individual and is structured to sense radiation as it emanates from the target area of the individual.

In many instances, the result of temperature measurement in a non-invasive manner, as set forth above, is more accurate as compared to the contact-type devices prevalent in previous years. This is due in part to the fact that the influence of the temperature sensing element on the target is smaller, particularly in the case when the heat capacity of the target is relatively small. In cases of medical examination, it may be troublesome, impractical or in certain instances even dangerous to utilize a contact-type of heat measuring device, based on the location or target area on the patient from which the temperature is to be taken. Accordingly, the development of thermometers and medical devices for the collecting of medical data including, but not limited to, temperatures has significantly advanced. As such it is now recognized that the non-invasive temperature acquiring devices may be preferable for many temperature acquiring applications.

Further, the use of infrared technology in acquiring temperatures by the non-invasive technique may also be preferred in many instances. In prior years, infrared thermometers frequently contemplated the insertion of a probe portion thereof into the ears or other appropriate orifices of the patient. However, advancement in infrared thermometers and the technology associated therewith allow for accurate readings of temperature. Even with such advancements in infrared thermometers, there are still certain limitations which are not available with conventional and/or commercially available devices.

Document GB2380791A discloses an instrument for medical diagnosis of a body.

Document US2002016553A1 discloses a method and a device for managing body temperature.

Accordingly, there is a need in this area for an improved, proposed non-invasive medical data collecting assembly which is capable of storing temperatures or other medical data collected from a plurality of patients or from one patient successively over a period of time. As such, a new and proposed medical data collecting assembly should incorporate adequate memory capabilities for the storage of a significant number of collected temperatures or other medical data, as well as the ability to easily and quickly access the stored temperatures. Further, such a proposed and improved medical data collecting assembly should be capable of identifying each of the temperatures or like collected data with the individual patients and possibly include other required information such as time, date, etc. when the medical data was collected.

Finally, yet an additional feature of such an improved and proposed medical data collecting assembly should be the ability to download collected medical data to one or more additional, remotely located processing facilities. Such processing facilities may assume a wide variety of structural and operative features including a variety of computer facilities having significant storage capacity and the ability to communicate, on an interactive basis with the data collecting assembly as well as any one of a plurality of other remotely located processing facilities.

### SUMMARY OF THE INVENTION

This invention relates to a device structured to collect medical data from at least one patient but more practically a plurality of patients in a non-invasive manner. The medical data collected may include the temperature of the patient or patients but may also include additional and/or other information such as blood pressure, glucose levels, etc.

According to the invention an assembly structured to collect medical data from at least one individual is provided as defined in claim 1. Further developments of the invention are the subject of the dependent claims.

Moreover, the medical data collection assembly of the present invention includes a hand held device comprising a housing dimensioned and configured to be held and operated by a single hand of a user. When so used, the housing is disposed in a predetermined operative position relative to the patient being examined. As will be more specifically described hereinafter, the operative position comprises the hand-held housing being preferably disposed a predetermined spaced distance from a target area of the patient such as, but not limited to, the patient's forehead. Therefore, the hand-held housing of the device includes a plurality of interconnected or otherwise operatively associated components which cooperate to collect the predetermined medical data and store a plurality of readings representative of the data collected from one or a plurality of patients in a readily accessible manner.

In addition, a display assembly is mounted on the hand-held device and is interconnected or operatively associated to communicate with both a sensor assembly as well as a memory assembly. The sensor assembly is structured to determine and obtain the intended medical data and the memory assembly is structured to store the medical data, once obtained by the sensor assembly. A control assembly is disposed on the housing and is accessible and readily operable from an exterior thereof such as by the hand of a user serving to grip, support and position the housing relative to the patient. Therefore, the control assembly which may include a plurality of hand manipulated buttons, switches, etc. mounted on the exterior of the housing serve to activate, operate and/or accomplish interactive communication between the various components including, but not limited to the sensor assembly, memory assembly and display assembly.

Further, the control assembly, as well as the interactive communicative features of the sensor assembly and memory assembly in association with the display assembly, serves to activate the sensor assembly when the hand-held housing is in the aforementioned operative position in spaced, non-invasive relation to a patient. Further operation of the control assembly will serve to activate the sensor assembly facilitating the collection of the intended medical data. Substantially concurrently to such collection of data, the control assembly may be further operated to transfer the collected data directly to the display assembly. Alternatively the collected data may be automatically transferred and/or stored in the memory assembly. In either case, the display assembly is structured and interactively associated with both the sensor assembly and the memory assembly to display medical data which has been just collected from a patient or which has been stored and subsequently accessed from the memory assembly.

Moreover, the memory assembly is structured to store and permit selective access each of a predetermined number of collected data measurements such as, but not limited to, a plurality of temperatures obtained from a plurality of patients. In the alternative, a plurality of temperatures can be obtained from a single patient over a predetermined time and sequentially stored upon collection. Operation of the control assembly will serve to access any of the collected medical data stored in the memory assembly thereby facilitating observation or viewing thereof on the display assembly. Additional features of the control assembly being interactive with the memory assembly, is the entering of information data corresponding with each of the collected medical data inputs stored in the memory assembly. More specifically, "identification information" representative of the identity of each of the plurality of patients may be input by an appropriate user interface, preferably on the hand held device. As such, each medical data input, such as temperature, obtained from one or more patients may be directly associated with appropriate identification information such that the collected data may be readily identified and directly associated with a given patient. It is also to be noted that the user interface may enter additional and/or supplementary information, such as time of taking the temperature or other medical data collected as well as other appropriate information which may be used to more accurately evaluate the temperature or other medical data collected.

Another operative and structural feature of at least one preferred embodiment of the present invention is the inclusion of a docking station which is removably connected and/or disposed in supporting relation to the hand-held housing portion of the collecting assembly. In addition, the docking assembly may include a data transmitting assembly and be otherwise structured to be connected or disposed in communicating relation with the memory assembly of the hand-held housing. More specifically, when the housing is removably supported in an operative orientation on the docking assembly, there is an interactive communication between the data transmitting assembly associated with the docking assembly and the memory assembly associated with the hand-held housing. Such operative, interactive communication serves to effectively download the stored, collected medical data into the data transmitting assembly and/or any supplementary storage capability associated therewith. Alternatively, instead of downloading of the collected and stored medical data from the memory assembly into the data transmitting assembly, one embodiment of the present invention comprises the direct access and transmission of the stored data in the memory assembly by the data transmitting assembly to a remote processing facility. The interactive communication between the data transmitting assembly and the memory assembly may be accomplished by a hard wire connection, wireless connection, including the utilization of BlueTooth protocol or by other appropriate communication means.

Once the collected medical data is accessed or downloaded from the memory assembly of the hand-held housing, the data transmitting assembly is further structured to communicate the collected medical data to a remote site for storage and/or further processing. As such, the data transmitting assembly may be structured for one way or two way communication with a remote processing facility such as, but not limited to, a remote computer. The remote processing facility may therefore be capable of additional, larger capacity storage, selective accessing and review, further transmission of the received, collected medical data to additional remote processing facilities and/or other processing procedures.

At the same time it is emphasized that the memory assembly associated with the hand-held housing portion of the collecting assembly of the present invention may still maintain the plurality of collected medical data stored therein after being accessed by the data collecting assembly. Accordingly, the collected medical data may be individually accessed and displayed on the aforementioned display assembly even after transmission of such medical data by the data transmitting assembly has taken place.

These and other objects, features and advantages of the present invention will become clearer when the drawings as well as the detailed description are taken into consideration.

### BRIEF DESCRIPTION OF THE DRAWINGS

For a fuller understanding of the nature of the present invention, reference should be had to the following detailed description taken in connection with the accompanying drawings in which:
Figure 1 is a rear perspective view of the medical data collecting assembly of the present invention.
Figure 2 is a front perspective view of the embodiment of Figure 1.
Figure 3 is a front view of the embodiment of Figures 1 and 2.
Figure 4 is a rear view of the embodiment of Figures 1-3.
Figure 5 is a schematic representation of various operative components associated primarily, but not exclusively, with the hand-held housing portion of the assembly of the embodiments of Figures 1-4.
Figure 6 is a schematic view of the various operative components associated with a docking assembly which is part of the embodiments of Figures 1-4.

Like reference numerals refer to like parts throughout the several views of the drawings.

### DETAILED DESCRIPTION OF THE PREFERRED EMBODIMENT

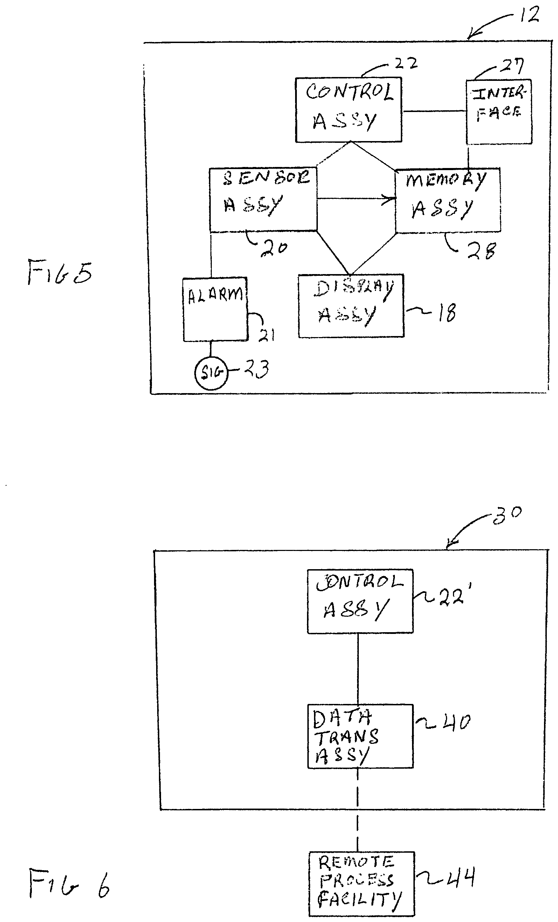

As represented in the accompanying drawings, the present invention is directed to a non-invasive medical data collecting assembly generally indicated as 10. Moreover, the collecting assembly 10 includes a hand held device having a housing generally indicated as 12 structured to be hand operated during the data collecting procedure. Structural details of the housing 12 include a handle portion generally indicated as 14 and a head or containment portion generally indicated as 16 on which a display assembly 18 and a sensor assembly 20 are disposed. As should be apparent, the operative components, circuitry, etc. of both the display assembly 18 and the sensor assembly 20 are contained within an at least partially hollow interior of the head portion 16 of the device or housing 12. As such these components are interconnected or otherwise operatively associated so as to establish interactive communication therebetween as will be explained in greater detail with regard to the schematic representation of Figure 5.

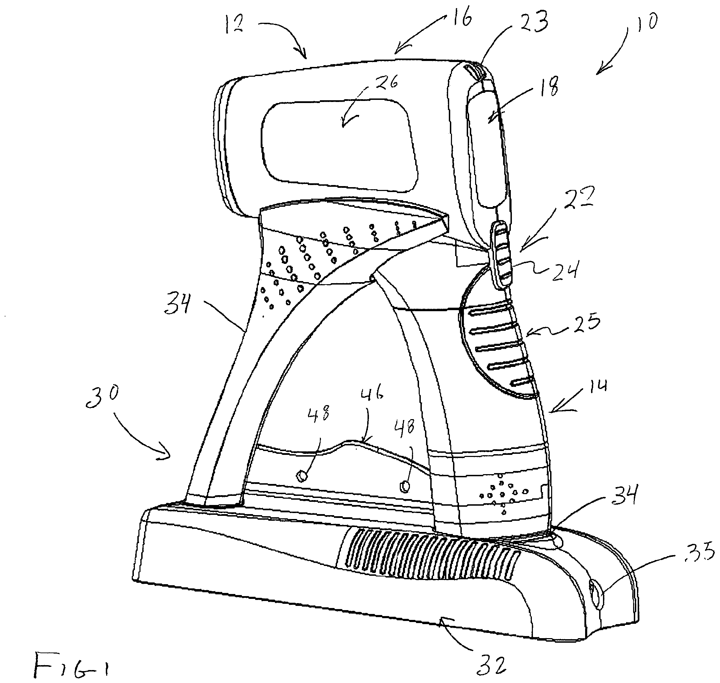

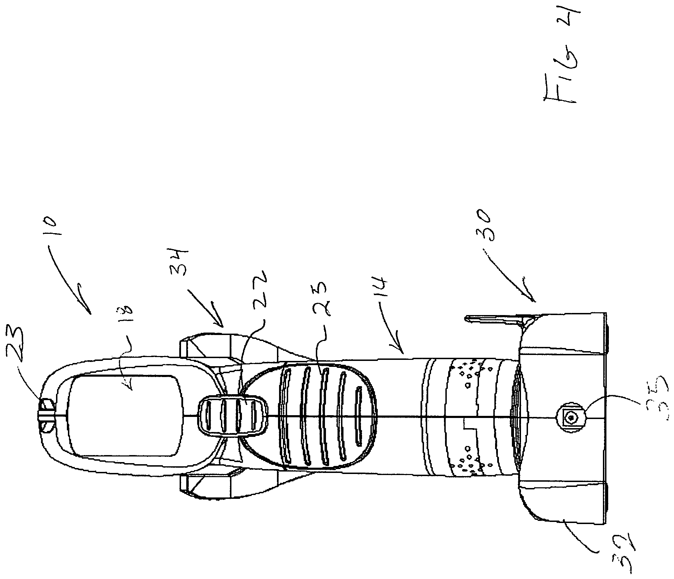

A control assembly is generally referred to, at least in part, as 22 and includes one or a plurality of control buttons or other control structures 24, 24, etc, which may be manipulated by the hand and/or fingers of the user which serve to grip and support the housing 12. A plurality of the control buttons 24, 25, etc, are represented in Figures 1 and 4. However, a number of other operative buttons or control members may also define at least a portion of the control assembly 22 and may be located on the exterior of the housing 12 in a readily accessible position on the housing 12. By way of example, such additional buttons or control members may be located on a panel area generally indicated as 26. Such additional control buttons or like structures, while not shown for purposes of clarity, may vary in number, size, configuration and structure to facilitate effective operation of the device or housing 12 of the assembly 10.

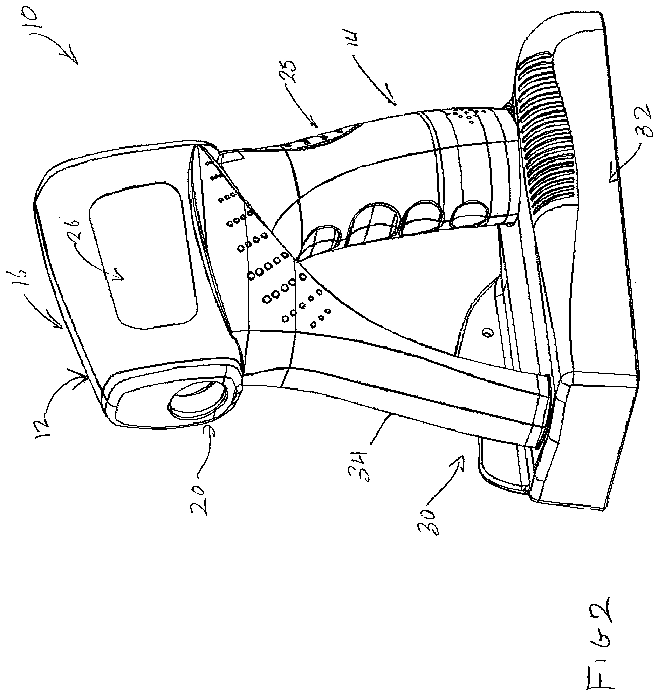

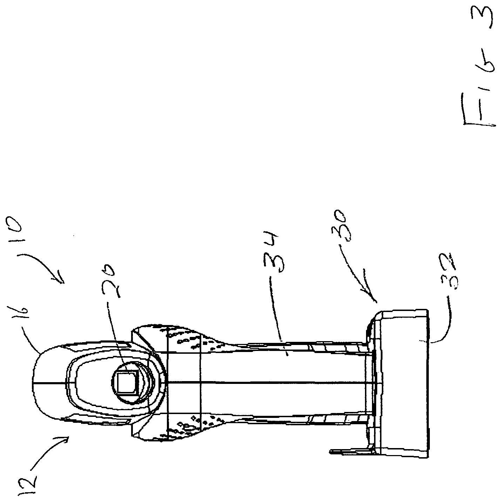

Another feature of the data collecting assembly 10 includes a docking station generally indicated as 30. The docking station 30 preferably includes a base portion 32 and a supporting yoke, pedestal or like member 34 which serves to support and facilitate engagement with the device or housing 12 in the manner represented in Figures 1-4. In addition, the base 32 includes an input or receiving terminal 34 which may be structured to establish operative, interactive communication between a data transmitting assembly, generally indicated as 40 in Figure 6, and one or more of the various operative components of the hand-held device or housing portion 12. Such operative components which are structured to establish interactive communication with the data transmitting assembly 40 include the control assembly 22, sensor assembly 20, memory assembly 28 and possibly the display assembly 18. As will also be explained in greater detail hereinafter with specific regard to the operational features and characteristics of the assembly 10, the memory assembly 28 is also included within the collecting assembly 10 and may be directly associated with the hand-held housing 12. Base 32 of the docking station 30 also includes an input socket or like junction port 35 for receipt of a power cord and/or communication line or both.

Accordingly, as represented in the various Figures, the hand-held housing 12 is removably supported and/or connected to the base 32 of the docking assembly 30. When in such a docked position or orientation the various components of the housing 12 are operatively associated by hardwire connection or wireless interface with the various operative components of the docking assembly 30. Further, the operative components of the hand-held housing 12 may be powered by a battery or battery pack which may be replaceable or which may be rechargeable, such as when the housing 12 is in its docked orientation as represented in Figures 1-4.

Additional structural and operative features will be explained with primary reference to Figures 5 and 6, schematically representing the interactive, operative association of the various components associated with both the hand-held device or housing portion 12 of the assembly 10 as well as the docking station 30 of the assembly 10. More specifically, the control assembly 22 is operable through hand manipulation of the operating or control buttons 24, 25, etc. on the housing 12. When manipulated these control buttons serve to activate and/or operate the operative components at least associated with the housing 12. As set forth above, the operative components associated with the housing 12 include the sensor assembly 20 which is preferably operable through the provision of infrared technology such as, but not limited to, the use of Heimann infrared technology. This infrared technology is especially conceived to measure the bodies' temperature instantly and is adaptable for the obtaining or collection of other medical data including glucose levels, blood pressure, etc.

As such, the control assembly is operatively associated with or connected to the sensor assembly 20 as well as the memory assembly 28. The memory assembly 28 is capable of storing collected medical data such as, but not limited to, a predetermined number or plurality of temperature readings from one and/or a plurality of patients. As is schematically represented in Figure 5, the sensor assembly 20 is linked for interactive communication with the memory assembly 28. Therefore, the determination or collection of the intended medical data or temperature, when achieved, is transferred to the memory assembly 28 for storage. Further, the display assembly 18 is interconnected to both the sensor assembly 20 and the memory assembly 28 and may be activated either independently or automatically by hand manipulation of the control assembly 22. More specifically, upon the hand-held housing 12 being disposed in a preferred or predetermined operative position, in spaced relation to a target area of a patient such as the forehead, the intended medical data or temperature will be collected through operation of the sensor assembly. Such collected medical data will be transferred to memory assembly and be concurrently displayed for observation on the display assembly 18.

The user of the hand-held housing 12 may eventually pass on to a plurality of other patients and sequentially obtain the intended medical data, such as the temperatures, therefrom. Each collected temperature reading or other intended medical data is successively stored for independent access in the memory assembly 28. Other manipulation or operation of the control assembly 22 may serve to access the memory assembly 28 and in particular call for any of the plurality of previously collected temperature readings or other medical data by direct access or by incorporating a scrolling feature in the control assembly 22. Any accessed medical data which has been collected can then be displayed on the display assembly 18 for observation.

Further, the memory assembly 28 may be directly associated with a user interface 27 such as a plurality of input buttons which may be mounted at a convenient location on the hand-held housing 12 such as on the panel 26. Such interface 27 allows the input of identifying information so as to correspond each temperature reading or other collected medical data with a specific patient. Such identifying information may also involve other supplementary information such as the time or date when the temperature or other medical data was collected. Accordingly, proper operation of the control assembly through hand manipulation of the various control structures 24, 25, etc. on the hand-held housing 12 will serve to access any of the previously collected temperature readings or medical data as well as the identifying information associated therewith.

Other features associated with the hand-held device or housing 12 include an alarm assembly generally indicated as 21 which may be automatically activated upon the sensor assembly 20 determining that the temperature of the patient is outside of acceptable parameters. More specifically, when the sensor assembly 20 determines a temperature of, by way of example only, 101°F or more, the alarm assembly 21 may be automatically activated. The activation of the alarm assembly 21 may be evidenced by an appropriate icon or other display on the display assembly 18 or alternatively by a flashing light or audio indicator 23 appropriately positioned on the hand-held housing 12.

As set forth above, the medical data collecting assembly 10 also includes a docking station 30 as represented in Figures 1-4 and as schematically represented in Figure 6. The docking assembly 30 is removably connected to and serves to support the hand-held housing 12 such as by additional support by the upwardly protruding yoke or pedestal 34. The opposite or free end of the handle 14 is then removably inserted or disposed within the inlet or receiving socket 34 so as to facilitate data transfer between the memory assembly 28 and a data transmitting assembly 40, the latter being operatively associated with the docking station 30.

For purposes of clarity, a control assembly in Figure 6 is represented as 22' and may be associated with the control assembly 22 operatively associated with the hand-held device 12. However, when the housing 12 is connected to the docking station 30, the control assembly 22 and the control assembly 22' may in fact represent the same structure and operative function so as to facilitate data transfer from the memory assembly 28 to the data transmitting assembly 40, such as by manipulation of the control members 24, 25, etc. It is again emphasized that linking communication may be accomplished by a hardwire connection or wireless and/or BlueTooth capabilities between the hand-held housing 12 and the docking station 30 and more specifically the memory 28 and the data transmitting assembly 40.

The data transmitting assembly 40 may be structured to include either one way or two way communication and as such may be in the form of a wireless and/or hardwire transmitter or transceiver. In either of these structural and operative modifications, the medical data collected and stored within the memory assembly 28 may be transferred in whole or in part to the data transmitting assembly 40 through proper operation and activation of the control assembly 22, 22'. However, the interactive communication between the memory assembly 28 and the data transmitting assembly 40 is such that the collected data stored in the memory assembly 28 may be efficiently downloaded to the data transmitting assembly 40 or transmitted directly to a remote processing facility 44 through the data transmitting assembly 40. At the same time all medical data which has been collected and stored in the memory assembly 28 may remain there to facilitate quick and efficient access by a user of the hand held device 12 even after the collected medical data has been transmitted by the data transmitting assembly 40.

The data transmitting assembly 40 through appropriate wired or wireless transmission is capable of transmitting, transferring or uploading the collected medical data from the memory assembly 28 to the remote processing facility 44. Further, the remote processing facility 44 may be located in the same generally vicinity as the medical data collecting assembly 10 or alternatively may be located a significant, remote distance therefrom. Further, the remote processing facility 44 may be in the form of any one of a plurality of different computer facilities capable of permanent and/or larger capacity storage. Moreover, the remote processing facility 44 may be capable of further communication with other processing assemblies so as to distribute the collected medical data in a timely and efficient manner for further observation and evaluation.

Yet other features associated with the medical data collecting assembly 10 as represented in Figures 1-4 is a mounting structure 46 having through holes 48. Holes 48 may be dimensioned, disposed and configured to receive any type of appropriate connector for mounting the docking assembly 30 on an appropriate wall or like supporting surface. Alternatively, the under surface portion of the base 32 of the docking station 30 may be mounted on a horizontal support surface if preferred

Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims.

## Claims

1. An assembly (10) structured to collect medical data from at least one individual, said assembly (10) comprising:
- a hand-held device comprising a housing (12) disposable in an operative position relative to the patient, the housing (12) including a handle portion (14) and a head portion (16), said housing (12) being dimensioned and configured to be held and operated by a single hand of a user,
- a sensor assembly (20) disposed on said head portion (16) of the housing (12) and structured to non-invasively and instantly determine the medical data while said housing (12) is in said operative position,
- said operative position comprising said housing (12) disposed within a predetermined spaced distance from the patient,
- a memory assembly (28) cooperatively structured with said sensor assembly (20) to concurrently store collected medical data of a plurality of patients, said memory assembly (28) being associated with an interface (27) which allows the input of identifying information in respect of said plurality of patients,
- a display assembly (18) disposed on said head portion (16) of the housing (12) and structured to view collected medical data, said display assembly (18) being structured and interactively associated with both the sensor assembly (20) and the memory assembly (28) to display medical data which has been just collected from a patient or which has been stored and subsequently accessed from the memory assembly (28),
- a control assembly (21) at least partially disposed on said housing (12) and structured to access any of the medical data stored in said memory assembly (28) , said control assembly (22) being operatively associated with the sensor assembly (20) and the memory assembly (28) and including one or a plurality of operating or control buttons (24, 25) which may be manipulated by the hand and/or fingers or the user which serve to grip and support the handle portion (14) of the housing (12) , the display assembly (18) being activated either independently or automatically by hand manipulation of the control assembly (21),
- a docking station (30) operatively and removably connected to said housing (12), said docking station (30) including a base portion (32) and an upwardly protruding supporting member (34) which serves to support and facilitate engagement with the housing of the hand-hold device and,
- a medical data transmitting assembly (40) operatively associated with the docking station (30) and cooperatively structured with said memory assembly (28) to communicate the medical data maintained in said memory assembly (28) to a remote processing facility (44), through the data transmitting assembly (40), the collected data stored in the memory assembly (28) being downloaded to the data transmitting assembly (40) or, transmitted directly to a remote processing facility (44).

2. An assembly (10), as recited in claim 1, wherein said medical data transmitting assembly (40) is further structured for wireless communication between said memory assembly (28) and said remote processing facility (44).

3. An assembly (10), as recited in claim 1, wherein said medical data transmitting assembly (40) is at least partially disposed on said docking station (30).

4. An assembly (10), as recited in claim 1, wherein said sensor assembly (20) comprises infrared capabilities and is structured to determine medical data at least partially defined by a temperature of the patient.

5. An assembly (10), as recited in any of claims 1 to 4, wherein said sensor assembly (20) is structured to determine a temperature of the patient when disposed in said operative position, said operative position defined by a predetermined spaced distance from the target area defined by the forehead of the patient.

6. An assembly (10), as recited in any of claims 1 to 5, further comprising an alarm assembly (21) operatively associated with at least said sensor assembly (20) and structured to generate an alarm upon the medical data exceeding predetermined parameters.

7. An assembly (10), as recited in any of claims 1 to 6, wherein said control assembly (22) is further structured to access identifying information stored in the memory assembly (28) and correlate the collected medical data with a corresponding one of the plurality of patients.

8. An assembly (10), as recited in claim 7, wherein said control assembly (22) comprises search capabilities operatively associated with said memory assembly (28) and structured to facilitate selective access of collected medical data and identification information of any of the plurality of patients.

9. An assembly (10), as recited in claim 1, wherein said data transmitting assembly (40) is structured to communicate collected medical data and identification information of selected patients to the remote processing facility (44).

10. An assembly (10), as recited in claim 9, wherein said memory assembly (28) and said control assembly (22) are cooperatively structured to maintain the medical data and the identification information in said memory assembly (28) subsequent to transmission to the remote processing facility (44).

11. An assembly (10), as recited in any of claims 1 to 10, wherein the handle portion (14) has an end connected to the head portion (16) and an opposite free end, the base portion (32) of the docking station (30) having an inlet (34) and being operatively and removably connected to the free end of the handle portion (14) removably inserted or disposed within said inlet (34).

12. An assembly (10), as recited in claim 11, wherein the upwardly protruding supporting member (34) serves to support and facilitate engagement with the head portion (16).

13. An assembly (10), as recited in any of claims 1 to 12, wherein the handle portion (14) and the head portion (16) are configured so as to confer to the housing (12) an overall shape of a hair dryer, the head portion (16) of which is higher than wider, the height of the housing (12) being taken along a direction extending from a free end of the handle portion (14) to the head portion (16).

## Patentansprüche

1. Anordnung (10), die dazu ausgestaltet ist, medizinische Daten von mindestens einer Person zu erheben, wobei die Anordnung (10) Folgendes umfasst:
- eine handgehaltene Vorrichtung, die ein Gehäuse (12) umfasst, das in einer Betriebsposition relativ zum Patienten anordenbar ist, wobei das Gehäuse (12) einen Griffabschnitt (14) und einen Kopfabschnitt (16) umfasst, wobei das Gehäuse (12) dazu dimensioniert und ausgebildet ist, von einer einzelnen Hand eines Benutzers gehalten und betrieben zu werden,
- eine Sensoranordnung (20), die auf dem Kopfabschnitt (16) des Gehäuses (12) angeordnet und dazu ausgestaltet ist, nicht invasiv und augenblicklich die medizinischen Daten zu bestimmen, während sich das Gehäuse (12) in der Betriebsposition befindet,
- wobei die Betriebsposition umfasst, dass das Gehäuse (12) innerhalb eines vorbestimmten beabstandeten Abstands vom Patienten angeordnet ist,
- eine Speicheranordnung (28), die zusammenwirkend mit der Sensoranordnung (20) ausgestaltet ist, um erhobene medizinische Daten einer Mehrzahl von Patienten gleichzeitig zu speichern, wobei die Speicheranordnung (28) mit einer Schnittstelle (27) verknüpft ist, die die Eingabe von Identifikationsinformationen in Bezug auf die Mehrzahl von Patienten ermöglicht,
- eine Anzeigeanordnung (18), die auf dem Kopfabschnitt (16) des Gehäuses (12) angeordnet und dazu ausgestaltet ist, erhobene medizinische Daten anzusehen, wobei die Anzeigeanordnung (18) dazu ausgestaltet und sowohl mit der Sensoranordnung (20) als auch der Speicheranordnung (28) interaktiv verknüpft ist, um medizinische Daten anzuzeigen, die gerade von einem Patienten erhoben wurden oder die gespeichert wurden und auf die anschließend von der Speicheranordnung (28) zugegriffen wurde,
- eine Steueranordnung (21), die zumindest teilweise auf dem Gehäuse (12) angeordnet und dazu ausgestaltet ist, auf beliebige der in der Speicheranordnung (28) gespeicherten medizinischen Daten zuzugreifen, wobei die Steueranordnung (22) mit der Sensoranordnung (20) und der Speicheranordnung (28) betriebsmäßig verknüpft ist und eine oder eine Mehrzahl von Betriebs- oder Steuertasten (24, 25) umfasst, die durch die Hand und/oder Finger des Benutzers betätigt werden können, die dazu dienen, den Griffabschnitt (14) des Gehäuses (12) zu greifen und zu tragen, wobei die Anzeigeanordnung (18) entweder unabhängig oder automatisch durch eine Handbetätigung der Steueranordnung (21) aktiviert wird,
- eine Andockstation (30), die betriebsmäßig und entfernbar mit dem Gehäuse (12) verbunden ist, wobei die Andockstation (30) einen Basisabschnitt (32) und ein nach oben hervorstehendes Tragelement (34) umfasst, das dazu dient, das Gehäuse der handgehaltenen Vorrichtung zu tragen und einen Eingriff damit zu erleichtern, und
- eine Anordnung (40) zur Übertragung von medizinischen Daten, die mit der Andockstation (30) betriebsmäßig verknüpft ist und zusammenwirkend mit der Speicheranordnung (28) ausgestaltet ist, um die in der Speicheranordnung (28) vorgehaltenen medizinischen Daten über die Anordnung (40) zur Übertragung von Daten an eine entfernte Verarbeitungsanlage (44) zu kommunizieren, wobei die in der Speicheranordnung (28) gespeicherten erhobenen Daten zu der Anordnung (40) zur Übertragung von Daten heruntergeladen oder direkt an eine entfernte Verarbeitungsanlage (44) übertragen werden.

2. Anordnung (10) nach Anspruch 1, wobei die Anordnung (40) zur Übertragung von medizinischen Daten ferner für eine drahtlose Kommunikation zwischen der Speicheranordnung (28) und der entfernten Verarbeitungsanlage (44) ausgestaltet ist.

3. Anordnung (10) nach Anspruch 1, wobei die Anordnung (40) zur Übertragung medizinischer Daten zumindest teilweise auf der Andockstation (30) angeordnet ist.

4. Anordnung (10) nach Anspruch 1, wobei die Sensoranordnung (20) Infrarotfähigkeiten umfasst und dazu ausgestaltet ist, medizinische Daten zu bestimmen, die zumindest teilweise durch eine Temperatur des Patienten definiert sind.

5. Anordnung (10) nach einem der Ansprüche 1 bis 4, wobei die Sensoranordnung (20) dazu ausgestaltet ist, eine Temperatur des Patienten zu bestimmen, wenn sie in der Betriebsposition angeordnet ist, wobei die Betriebsposition durch einen vorbestimmten beabstandeten Abstand von dem durch die Stirn des Patienten definierten Zielbereich definiert ist.

6. Anordnung (10) nach einem der Ansprüche 1 bis 5, ferner umfassend eine Alarmanordnung (21), die mit mindestens der Sensoranordnung (20) betriebsmäßig verknüpft und dazu ausgestaltet ist, einen Alarm zu generieren, wenn die medizinischen Daten vorbestimmte Parameter überschreiten.

7. Anordnung (10) nach einem der Ansprüche 1 bis 6, wobei die Steueranordnung (22) ferner dazu ausgestaltet ist, auf in der Speicheranordnung (28) gespeicherte Identifikationsinformationen zuzugreifen und die erhobenen medizinischen Daten mit einem entsprechenden der Mehrzahl von Patienten zu korrelieren.

8. Anordnung (10) nach Anspruch 7, wobei die Steueranordnung (22) Suchfähigkeiten umfasst, die mit der Speicheranordnung (28) betriebsmäßig verknüpft und dazu ausgestaltet sind, einen gezielten Zugriff auf erhobene medizinische Daten und Identifikationsinformationen eines beliebigen der Mehrzahl von Patienten zu erleichtern.

9. Anordnung (10) nach Anspruch 1, wobei die Anordnung (40) zur Übertragung von Daten dazu ausgestaltet ist, erhobene medizinische Daten und Identifikationsinformationen ausgewählter Patienten an die entfernte Verarbeitungsanlage (44) zu kommunizieren.

10. Anordnung (10) nach Anspruch 9, wobei die Speicheranordnung (28) und die Steueranordnung (22) zusammenwirkend ausgestaltet sind, um die medizinischen Daten und die Identifikationsinformationen anschließend an eine Übertragung an die entfernte Verarbeitungsanlage (44) in der Speicheranordnung (28) vorzuhalten.

11. Anordnung (10) nach einem der Ansprüche 1 bis 10, wobei der Griffabschnitt (14) ein mit dem Kopfabschnitt (16) verbundenes Ende und ein gegenüberliegendes freies Ende aufweist, wobei der Basisabschnitt (32) der Andockstation (30) einen Eingang (34) aufweist und betriebsmäßig und entfernbar mit dem freien Ende des Griffabschnitts (14) verbunden ist, das entfernbar in den Eingang (34) eingesetzt oder darin angeordnet ist.

12. Anordnung (10) nach Anspruch 11, wobei das nach oben hervorstehende Tragelement (34) dazu dient, den Kopfabschnitt (16) zu tragen und einen Eingriff damit zu erleichtern.

13. Anordnung (10) nach einem der Ansprüche 1 bis 12, wobei der Griffabschnitt (14) und der Kopfabschnitt (16) dazu ausgelegt sind, dem Gehäuse (12) eine Gesamtform eines Haartrockners zu verleihen, wobei der Kopfabschnitt (16) davon höher als breit ist, wobei die Höhe des Gehäuses (12) entlang einer Richtung genommen ist, die sich von einem freien Ende des Griffabschnitts (14) zu dem Kopfabschnitt (16) erstreckt.

## Revendications

1. Ensemble (10) structuré pour collecter des données médicales à partir d'au moins un individu, ledit ensemble (10) comprenant :
- un dispositif tenu en main comprenant un boîtier (12) pouvant être disposé dans une position opérationnelle relativement au patient, le boîtier (12) incluant une partie poignée (14) et une partie tête (16), ledit boîtier (12) étant dimensionné et configuré pour être tenu et utilisée par une seule main d'un utilisateur,
- un ensemble capteur (20) disposé sur ladite partie tête (16) du boîtier (12) et structuré pour déterminer, de façon non effractive et instantanément, les données médicales alors que ledit boîtier (12) est dans ladite position opérationnelle,
- ladite position opérationnelle comprenant ledit boîtier (12) disposé au sein d'une distance espacée prédéterminée par rapport au patient,
- un ensemble mémoire (28) coopérativement structuré avec ledit ensemble capteur (20) pour stocker simultanément des données médicales collectées d'une pluralité de patients, ledit ensemble mémoire (28) étant associé à une interface (27) qui permet l'entrée d'informations d'identification en ce qui concerne ladite pluralité de patients,
- un ensemble écran d'affichage (18) disposé sur ladite partie tête (16) du boîtier (12) et structuré pour voir des données médicales collectées, ledit ensemble écran d'affichage (18) étant structuré et interactivement associé à la fois à l'ensemble capteur (20) et à l'ensemble mémoire (28) pour afficher des données médicales qui ont juste été collectées à partir d'un patient ou qui ont été stockées et auxquelles l'accès dans l'ensemble mémoire (28) a par la suite été obtenu,
- un ensemble commande (21) au moins partiellement disposé sur ledit boîtier (12) et structuré pour accéder à de quelconques des données médicales stockées dans ledit ensemble mémoire (28), ledit ensemble commande (22) étant opérationnellement associé à l'ensemble capteur (20) et l'ensemble mémoire (28) et incluant un ou une pluralité de boutons opérationnels ou de commande (24, 25) qui peuvent être manipulés par la main et/ou les doigts ou l'utilisateur qui servent à saisir et supporter la partie poignée (14) du boîtier (12), l'ensemble écran d'affichage (18) étant activé soit indépendamment soit automatiquement par manipulation de l'ensemble commande (21),
- une station d'accueil (30) reliée opérationnellement et de façon amovible audit boîtier (12), ladite station d'accueil (30) incluant une partie base (32) et un élément support (34), faisant saillie vers le haut, qui sert à supporter et faciliter l'entrée en prise avec le boîtier du dispositif tenu en main et,
- un ensemble transmetteur de données médicales (40) opérationnellement associé à la station d'accueil (30) et coopérativement structuré avec ledit ensemble mémoire (28) pour communiquer les données médicales maintenues dans ledit ensemble mémoire (28) à une installation de traitement à distance (44), par l'intermédiaire de l'ensemble transmetteur de données (40), les données collectées stockées dans l'ensemble mémoire (28) étant téléchargées dans l'ensemble transmetteur de données (40) ou transmises directement à une installation de traitement à distance (44).

2. Ensemble (10)selon la revendication 1, dans lequel ledit ensemble transmetteur de données médicales (40) est en outre structuré pour communication sans fil entre ledit ensemble mémoire (28) et ladite installation de traitement à distance (44).

3. Ensemble (10)selon la revendication 1, dans lequel ledit ensemble transmetteur de données médicales (40) est au moins partiellement disposé sur ladite station d'accueil (30).

4. Ensemble (10)selon la revendication 1, dans lequel ledit ensemble capteur (20) comprend des capacités infrarouges et est structuré pour déterminer des données médicales au moins partiellement définies par une température du patient.

5. Ensemble (10)selon l'une quelconque des revendications 1 à 4, dans lequel ledit ensemble capteur (20) est structuré pour déterminer une température du patient lorsqu'il est disposé dans ladite position opérationnelle, ladite position opérationnelle étant définie par une distance espacée prédéterminée par rapport à la zone cible définie par le front du patient.

6. Ensemble (10)selon l'une quelconque des revendications 1 à 5, comprenant en outre un ensemble alarme (21) opérationnellement associé au moins audit ensemble capteur (20) et structuré pour générer une alarme lorsque les données médicales dépassent des paramètres prédéterminés.

7. Ensemble (10)selon l'une quelconque des revendications 1 à 6, dans lequel ledit ensemble commande (22) est en outre structuré pour accéder à des informations d'identification stockées dans l'ensemble mémoire (28) et corréler les données médicales collectées avec un correspondant de la pluralité de patients.

8. Ensemble (10)selon la revendication 7, dans lequel ledit ensemble commande (22) comprend des capacités de recherche opérationnellement associées audit ensemble mémoire (28) et structurées pour faciliter l'accès sélectif à des données médicales collectées et des informations d'identification d'un quelconque de la pluralité de patients.

9. Ensemble (10)selon la revendication 1, dans lequel ledit ensemble transmetteur de données (40) est structuré pour communiquer des données médicales collectées et des informations d'identification de patients sélectionnés à l'installation de traitement à distance (44).

10. Ensemble (10)selon la revendication 9, dans lequel ledit ensemble mémoire (28) et ledit ensemble commande (22) sont coopérativement structurés pour maintenir les données médicales et les informations d'identification dans ledit ensemble mémoire (28) suivant la transmission à l'installation de traitement à distance (44).

11. Ensemble (10)selon l'une quelconque des revendications 1 à 10, dans lequel la partie poignée (14) a une extrémité reliée à la partie tête (16) et une extrémité libre opposée, la partie base (32) de la station d'accueil (30) ayant une entrée (34) et étant reliée opérationnellement et de façon amovible à l'extrémité libre de la partie poignée (14) insérée ou disposée de façon amovible à l'intérieur de ladite entrée (34).

12. Ensemble (10)selon la revendication 11, dans lequel l'élément support (34), faisant saillie vers le haut, sert à supporter et faciliter l'entrée en prise avec la partie tête (16).

13. Ensemble (10)selon l'une quelconque des revendications 1 à 12, dans lequel la partie poignée (14) et la partie tête (16) sont configurées afin de conférer au boîtier (12) une forme générale d'un sèche-cheveux, dont la partie tête (16) est plus haute que large, la hauteur du boîtier (12) étant prise le long d'une direction s'étendant depuis une extrémité libre de la partie poignée (14) jusqu'à la partie tête (16).
